# EUROPEAN PATENT SPECIFICATION

(11) **EP 0 762 870 B1**
(45) Date of publication and mention of the grant of the patent: **11.09.2002**
(21) Application number: 95919296.4
(22) Date of filing: 31.05.1995
(51) Int. Cl.: A61K 9/127, A61K 9/50, C12N 15/88

(54) **VIROSOME-MEDIATED INTRACELLULAR DELIVERY OF THERAPEUTIC AGENTS**
INTRAZELLULÄRE VERABREICHUNG THERAPEUTISCHER SUSTANZENMITTELS VIROSOMEN
VIROSOMES COMME VECTEUR POUR INTRODUIRE DES AGENTS THERAPEUTIQUES A L'INTERIEUR DE CELLULES

(30) Priority: 31.05.1994 US 251469
(43) Date of publication of application: 19.03.1997
(73) Proprietor: INEX Pharmaceutical Corp., Vancouver B.C. V6P 6P2 (CA)
(72) Inventor: WILSCHUT, Jan, C., NL-9393 PG Garnwerd (NL); SCHERRER, Peter, Vancouver, British Columbia V6H 2T5 (CA); CHONN, Arcadio, Vancouver, British Columbia V6Z 1E1 (CA)
(74) Representative: Thul, Stephan
(86) International application number: CA9500321
(87) International publication number: WO95032706

(56) References cited:
- EP-A- 0 497 997
- WO-A-92/03162
- US-A- 5 292 524
- GENE, vol. 84, no. 2, December 1989 AMSTERDAM (NL), pages 429-438, XP 000083965 S. GOULD-FOGERITE ET AL. 'CHIMERASOME-MEDIATED GENE TRANSFER IN VITRO AND IN VIVO'
- GENE, vol. 63, no. 2, 1988 AMSTERDAM (NL), pages 321-330, T.I. TIKCHONENKO ET AL. 'TRANSFER OF CONDENSED VIRAL DNA INTO EUKARYOTIC CELLS USING PROTEOLIPOSOMES'
- JOURNAL OF CONTROLLED RELEASE, vol. 19, no. 1-3, March 1992 AMSTERDAM (NL), pages 269-274, XP 000261538 X. ZHOU ET AL. 'TARGETED DELIVERY OF DNA BY LIPOSOMES AND POLYMERS'

## Description

### Background Of The Invention

The present invention relates to virosome compositions for pharmaceutical use. More particularly, the present invention relates to compositions of virosomes, consisting of liposomes with a functionally reconstituted viral membrane fusion protein, such as the influenza virus hemagglutinin, contained in their membranes, combined with therapeutic agents encapsulated in the aqueous lumen of the virosomes and/or contained in the virosomal membrane. The viral protein mediates fusion of the virosomal membrane with a cellular membrane and, therefore, facilitates delivery of the therapeutic agents into the cell.

Liposomes have been considered promising vehicles for targeting and delivery of biologically or pharmacologically active substances to living cells both in vitro and *in vivo* (Gregoriadis, Liposome Technology, CRC Press, Boca Raton, Fl., 2d ed., 1993). However, liposomes have little potential to fuse with cells and thus, generally fail to provide appreciable delivery of encapsulated molecules to the cell cytoplasm. Attempts to circumvent the refractoriness of liposomes to fusion have included preparing liposomes with different lipid compositions. For example, the so-called pH-sensitive liposomes seek to exploit the primary mechanism of cellular uptake of liposomes via endocytosis. As the endosomal cell compartment is acidic (Mellman et al., Ann. Rev. Biochem., 55:663 (1986)), pH-sensitive liposomes are designed to disintegrate following endocytosis (see, e.g., Conner et al., Proc. Natl. Acad. Sci. USA, 81:1715 (1984); Wang and Huang, Proc. Natl. Acad. Sci. USA, 84:7851 (1987); Nair et al., J. Exp. Med., 175:609 (1992)). pH-sensitive liposomes are unlikely to actually fuse with the endosomal membranes, however. When the pH surrounding the liposome is below the value of the pKₐ of an ionizable compound in the liposomal membrane, the compound becomes protonated and loses its ability to stabilize the vesicles. Consequently, the liposomes disintegrate and release their contents into the lumen of the endosome. In the course of this process a small fraction of the encapsulated contents may gain access to the cell cytoplasm through a concomitant transient and local destabilization of the endosomal membrane (Chu et al., Pharmaceut. Res. 7:824 (1990)). However, in general the efficiency of cytoplasmic delivery is low (Chu et al., id.; Legendre and Szoka, Pharmaceut. Res. 9:1235 (1992)). In addition, it is uncertain whether pH-sensitive liposomes can effectively be applied *in vivo* as it has been reported that pH-sensitive liposome compositions that allow for a complete release of liposomal contents at low pH are inherently unstable in human serum at 37°C (Tari et al., in Liposome Technology, supra).

Other attempts to enhance liposomal cytoplasmic drug delivery employ membrane formulations including cationic lipids. These liposomes have been designed specifically for transferring nucleic acids to cells. The DNA or RNA is complexed to small unilamellar liposomes composed of dioleoylphosphatidylethanolamine and cationic lipids, such as *N*-[1-(2,3-dioleyloxy)propyl]*-N,N,N*-trimethylammonium chloride (DOTMA; Felgner et al., Proc. Natl. Acad. Sci. USA, 84:7413 (1987)), or DOTAP, DDAB, etc. These liposomes interact with the plasma membranes of cells allowing translocation of nucleic acids into the cytoplasm. It is also possible that the nucleic acid-liposome complexes are endocytosed and that the actual translocation of the nucleic acids to the cytosol occurs from within the acidic endosomes. The DOTMA-containing liposome formulation has become commercially available under the name Lipofectin™. Although it recently has been demonstrated that Lipofectin can successfully be applied to pulmonary tissue via inhalation (Hyde et al., Nature, 362:250 (1993)), it is uncertain whether cationic lipid formulations can be applied in vivo when injected into tissues or into the circulation.

EP-A-0 497 997 describes a phospholipid bi-layer vesicle comprising influenza hemagglutinin on the membrane and at least one pharmaceutically active drug. Influenza virosomes containing a vaccine are disclosed in WO 92/03162. US-A-5 292 524 concerns a composition consisting of a blood platelet, a liposome or reconstituted Sendai virus envelopes being incorporated into the interior of the blood platelet, and a diagnostic or therapeutic agent encapsulated within the liposome or reconstituted Sendai virus envelopes. GENE, 84 (1989) 429-438 and GENE, 63 (1988) 321-330 disclose the use of a virosome to introduce a nucleic acid into a cell. Journal of Controlled Release, 19 (1992) 269-274 describes the use of cationic liposomes to deliver a nucleic acid into a cell.

What are needed in the art are methods for efficient and reliable introduction of encapsulated therapeutic nucleic acids into the cytoplasm of cells. In such methods the induction of membrane fusion between the encapsulating vesicle and the cell membrane is imperative to achieve efficient delivery of the encapsulated nucleic acids to cells. Quite surprisingly, the present invention fulfills these and other related needs.

### Summary Of The Invention

The present invention provides pharmaceutical compositions for treatment of hosts. The compositions generally comprise a virosome having a membrane and an aqueous interior, wherein a viral membrane fusion protein, e.g., influenza hemagglutinin protein, and a cationic lipid suitable for complexing a nucleic acid to the virosome are contained in the membrane, and further comprising a therapeutic nucleic acid contained in the virosome and a pharmaceutically acceptable carrier. The therapeutic nucleic acid may be carried in the aqueous interior or in the membrane of the virosome. Generally, the hemagglutinin is derived from influenza A. Furthermore, the present invention concerns the use of said virosome in the preparation of a medicament for introducing a therapeutic nucleic acid into a cell in a host. The virosomes may be administered to the host by a variety of routes, including by parenteral, topical or inhalation administration.

Also provided are methods for preparing a composition for introducing a nucleic acid into a cell of a host. The methods comprise condensing the nucleic acid with polylysine to form nucleic acid particles, combining the nucleic acid particles with a mixture of a viral membrane fusion protein, lipid and detergent, and removing the detergent to form a virosome having a membrane and an aqueous interior, the viral membrane fusion protein being contained in the membrane, thereby passively encapsulating the nucleic acid particles in the interior of the virosome.

### Brief Description Of The Drawings

Fig. 1 illustrates that the fusion of reconstituted influenza virosomes with erythrocyte membranes is dependent on low pH.

Fig. 2 illustrates the fusion of influenza virosomes from within BHK cell endosomes as monitored by a decrease of pyrene excimer fluorescence and the blocking of fusion by NH₄Cl, an inhibitor of endosomal acidification.

Fig. 3 illustrates that delivery of diphtheria toxin A chain encapsulated in fusogenic virosomes induces complete inhibition of the cellular protein synthesis in BHK-21 cells, whereas free DTA or empty virosomes have no effect on protein synthesis, and that the effect of virosome-encapsulated DTA is blocked completely by NH₄Cl, or by pretreatment of the virosomes at low pH causing an irreversible inactivation of their fusion activity.

Fig. 4 illustrates the time course of gelonin delivery to BHK cells, as mediated by influenza virosomes fusing from within endosomes.

Fig. 5 depicts gelonin delivery to BHK-21 cells mediated by influenza virosomes fusing from within endosomes.

Fig. 6 demonstrates that influenza virosomes can fuse with the plasma membrane of BHK cells, thereby mediating intracellular delivery of encapsulated gelonin.

Fig. 7 illustrates titration of gelonin-mediated inhibition of protein synthesis to a level corresponding to a single virosome fusing per cell.

Fig. 8 shows the expression of β-Gal in transfected BHK cells as a function of % DODAC in the fusion protein TCS.

Fig. 9 shows the DNA binding capacity of the virosomes containing 30 mol % DODAC, where increasing amounts of ³H-pCMVβ-gal were added to virosomes and, after incubation and centrifugation, radioactivity determined in the pellet (●, ■) and in the supernatant (▲, ▼) of two independent experiments.

Fig. 10 shows the expression of β-Gal in transfected BHK cells as a function of DNA added per well complexed to virosomes.

### Description Of The Specific Embodiments

Liposomes having membrane-bound viral envelope fusion protein (referred to herein as "virosomes") are employed as carriers for the therapeutic nucleic acid. As explained in more detail below, the viral fusion protein facilitates membrane fusion between the virosome and cell membranes to release the therapeutic nucleic acid into the cell cytoplasm.

"Liposome", "vesicle" and "liposome vesicle" will be understood to indicate structures having lipid-containing membranes enclosing an aqueous interior. The structures may have one or more lipid membranes unless otherwise indicated, although generally the liposomes will have only one membrane. Such single-layered liposomes are referred to herein as "unilamellar". Multilayer liposomes are referred to herein as "multilamellar".

The virosomes present in the pharmaceutical compositions of the present invention have at least one viral fusion protein, such as influenza hemagglutinin, in the membranes of the liposomes. This structure typically requires insertion of the viral fusion protein in the liposome membrane during preparation, as generally described in Bron et al., Meth. Enzymol., 220:313-331 (1993) and Stegmann et al., EMBO J. 6:2651-2659 (1987). The virosomes can also be prepared from other viruses which have lipid bilayer envelopes, such as Semliki Forest virus containing the viral fusion protein E1-E2, vesicular stomatitis virus having the G protein as a membrane fusion protein, Sendai virus having the HN and F membrane fusion proteins, and others.

For preparing virosomes, the viral membrane fusion protein such as, e.g., hemagglutinin, is often purified from the corresponding virus, but it can also be produced by recombinant techniques. Purification of hemagglutinin from viral stocks is described in more detail below. Hemagglutinin from human strains of influenza A, influenza B, or influenza C, or animal (avian, swine, equine, and the like) influenza strains may be used to prepare the virosomes, although influenza A hemagglutinin is generally preferred. A wide variety of suitable virus stocks are generally available as a hemagglutinin source, such as may be available from the American Type Culture Collection (ATCC), Rockville MD, or other sources.

Influenza virus has a lipid bilayer envelope. The virions acquire this membrane as they bud from the plasma membrane of an infected host cell. Enveloped viruses, in general, utilize membrane fusion to introduce their genome into the cytoplasm of new host cells during subsequent rounds of infection (see, e.g., White, Ann. Rev. Physiol., 52:675-697 (1990)). This fusion reaction may either occur at the level of the host cell plasma membrane, or within acidic endosomes after uptake of intact virions through receptor-mediated endocytosis. During endocytic cellular infection, the target membrane for fusion of the viral envelope is the limiting membrane of the endosomal cell compartment.

Influenza membrane fusion capacity is activated only under mildly acidic conditions. Low-pH-dependent viruses, such as influenza virus, must utilize the endocytic route of cellular infection for exposure to the necessary acidic conditions, which they encounter in the lumen of the endosomes (Mellman et al., Ann. Rev. Biochem. 55:663-700 (1986)). Fusion at the plasma membrane is precluded by the strict pH dependence of their fusion activity. Infection of cells by low-pH-dependent viruses can be blocked by inhibitors of vacuolar acidification, such as chloroquine or NH₄Cl. In cultured cell systems influenza virus can be induced to fuse with the cell plasma membrane by a transient lowering of the pH in the extracellular medium.

The influenza virus membrane contains two major integral spike glycoproteins, hemagglutinin (HA) and neuraminidase (NA). The infectious entry of the virions into the host cell is mediated by hemagglutinin. First, HA binds to sialic-acid-containing receptors on the cell surface. Second, following the internalization of the virus particles into the endosomal cell compartment (Stegmann et al., Biochim. Biophys. Acta, 904:165-170 (1987)), the HA also triggers the fusion reaction with the endosomal membrane.

The HA spike, protruding some 13.5 nm from the viral surface, is a homotrimeric molecule. Each monomer consists of two disulfide-linked subunits: HA1 (47 kD) and HA2 (28 kD), which are generated from a single polypeptide chain, HA0 (75 kD), by posttranslational cleavage by a host-cell protease. The globular HA1 domains contain the sialic-acid binding pockets. The N-terminus of HA2, generated by the posttranslational cleavage of HA0, appears crucial for the expression of fusion activity of HA: Uncleaved HA0 is not fusion-active, while site-specific mutations within this region of the molecule severely affect the fusion activity of HA (Gething et al., J. Cell Biol., 102:11-23 (1986)). The N-terminus of HA2, the so-called "fusion peptide", is a conserved stretch of some 20 amino acid residues that are mostly hydrophobic in nature (White, supra). At neutral pH the fusion peptides are buried within the stem of the HA trimer about 3.5 nm from the viral surface. However, at low pH an irreversible conformational change in the HA results in their exposure (White and Wilson, J. Cell Biol., 105:2887-2896 (1987)).

Influenza virus envelopes, including the hemagglutinin, can be solubilized by treatment of virus particles with a detergent. Nonionic detergents having a relatively low critical micellar concentration (CMC) are generally used to solubilize the envelope membranes. Octaethyleneglycol mono(n-dodecyl)ether (C₁₂E₈) and Triton X-100 may be used for solubilization, although other nonionic detergents may also be employed.

One potential disadvantage of using low-CMC detergents for solubilization and reconstitution of viral envelopes is that they can not be easily removed from the system by, e.g., dialysis. Detergents with a relatively high CMC such as N-octyl-β-D-glucopyranoside (octyl glucoside; CMC of about 20 nM), may be used to solubilize influenza virus envelopes. However, fusogenic virosomes are not readily prepared by subsequent removal of the octyl glucoside detergent. During dialysis, the hemagglutinin appears to concentrate primarily in lipid-poor aggregates with a very limited aqueous space, while the viral lipid is recovered in protein-poor vesicles. Although these vesicles exhibit some HA-mediated membrane fusion activity, only a small fraction of the HA is recovered in these vesicles (Stegmann et al., supra).

To obtain virus for solubilization, influenza virus is grown to high titers on cultured cells (e.g., Madin-Darby Kidney cells, or MDCK) or in the allantoic cavity of 10-day-old embryonated chicken eggs. To purify the virus from the allantoic fluid the harvested allantoic fluid is centrifuged (e.g., at 1000 g for 15 min in the cold) to remove debris, after which the virus is sedimented from the supernatant (e.g., at 75,000 g for 90 min at 4°). The virus pellet is resuspended in buffer such as "HNE" (150 mM NaCl, 0.1 mM EDTA, and 5 mM HEPES, adjusted to pH 7.4) and subjected to sucrose gradient centrifugation (e.g., 10-60%, w/v, linear sucrose gradient in HNE at 100,000 g for 16 hr at 4°). The virus equilibrates as a single band at approximately 45% (w/v) sucrose. The band is collected, then frozen in small aliquots at -80°. Virus can also be purified by a one-step affinity column chromatography, which is particularly useful with virus which has been obtained from cell culture. The protein content of virus preparations can be determined according to Peterson, Anal. Biochem., 83:346 (1977), and the phospholipid content, after quantitative extraction of the lipids from a known amount of virus, determined according to Böttcher *et al.,* Anal. Chim. Acta, 24:203 (1961).

For solubilization of viral envelopes a detergent such as, e.g., C₁₂E₈ (Nikko Chemicals, Tokyo, Japan; Fluka, Buchs, Switzerland; or Calbiochem, San Diego, CA) is dissolved in HNE at a concentration of about 100 mM. BioBeads SM2 (Bio-Rad, Richmond, CA) or the like are washed with methanol and subsequently with water, according to Holloway, Anal. Biochem., 55:304 (1973), and stored under water. Just before use the beads are drained on filter paper and weighed. Sucrose solutions for gradient centrifugation are made in HNE on a weight per volume basis.

A representative method for producing the virosomes of the invention is now described, although it will be understood that the procedure can be subjected to modifications in various aspects without affecting the outcome. As described more fully below in the experimental section, influenza virus (the equivalent of about 1.5 µmol membrane phospholipid) is diluted in HNE and sedimented (e.g., for 30 min at 50,000 g in a Beckman Ti50 rotor) at 4°. HNE buffer containing detergent is added to the pellet (e.g., 0.7 ml of 100 mM C₁₂E₈) and the pellet resuspended and solubilization allowed to occur for another 15 min on ice. Subsequently, the viral nucleocapsid is removed by centrifugation (e.g., for 30 min at 85,000 g at 4°) and a small sample of the supernatant can be taken at this stage for protein and phospholipid analysis. Of the initial viral protein and phospholipid, 35% (representing almost all of the membrane protein) and over 90%, respectively, may be recovered in the supernatant. The supernatant (e.g., 0.63 ml) is transferred to a 1.5-ml Eppendorf vial containing pre-washed BioBeads SM2 (e.g., 180 mg, wet weight) and the supernatant gently mixed with the beads. An additional amount of BioBeads (e.g., 90 mg wet) is added and mixing continued. The formation of vesicular structures is indicated when the suspension becomes turbid. An alternative procedure for removing the detergent from small volumes is according to Lundberg et al., Biochim. Biophys. Acta 1149: 305 (1993). BioBeads are packed into a minicolumn and the preparation run through the column. A centrifugation procedure or applying negative pressure can be used to force the preparation through the column. The column procedure provides more flexibility in terms of the ratio of the amount of BioBeads used and the volume of the preparation. The virosome suspension is then centrifuged on a discontinuous sucrose gradient (e.g., 10-40% (w/v) for 90 min at 130,000 g at 4°), and the virosomes appear as a thin opalescent band and are collected from the interface between the two sucrose-containing layers.

Other lipids can also be added to the virosome membranes during preparation. Fusion activity of the virosomes is optimally maintained when lipids similar to those of viral origin or lipid mixtures which closely resemble the lipid composition of the viral envelope are added. These lipids comprise cholesterol and phospholipids such as phosphatidylcholine (PC), sphingomyelin (SPM), phosphatidylethanolamine (PE), and phosphatidylserine (PS). However, other phospholipids may also be added. These include, but are not limited to, phosphatidylglycerol (PG), phosphatidic acid (PA), cardiolipin (CL), and phosphatidylinositol (PI), with varying fatty acyl compositions and of natural and/or (semi)synthetic origin, and dicetyl phosphate. Ceramide and various glycolipids, such as cerebrosides or gangliosides, may also be added. Cationic lipids are also added, e.g., for concentrating nucleic acids in the virosomes and/or for facilitating virosome-mediated delivery of nucleic acids to cells. These include DOTMA, DOTAP (N-[1-(2,3-dioleoyloxy)propyl] - N,N,N - trimethylammonium chloride), DODAC (N,N - dioleyl - N,N, dimethylammonium chloride), DDAB and stearylamine or other aliphatic amines and the like. DODAC is a preferred cationic lipid for complexing nucleic acids to the virosome and the ensuing delivery of nucleic acids to cells. Particularly preferred concentrations of DODAC range from 25-45% (mol % of total phospholiphids in the virus), more preferably 30-40%, and most preferably about 30% for the delivery of a nucleic acid such as DNA or antisense RNA to a cell. Additional lipids which may be suitable for use in the virosomes of the present invention are well known to persons of skill in the art. Nucleic acids such as oligonucleotides and DNA can also be encapsulated in virosomes after condensation with polylysine to form particles that are then enclosed within a virosome for delivery to a cell rather than being complexed to it, thereby minimizing, if desired, the use of a cationic lipid. Furthermore, encapsulated DNA is protected from DNase degradation.

Typically, in a virosome preparation procedure involving additional lipids, the additional lipids are dried from a mixed solution in chloroform/methanol to a film at the bottom of a tube by evaporation of the solvent and subsequent exposure to vacuum for 1 h. Then the supernatant fraction obtained after solubilization of the viral envelope in detergent (e.g., C₁₂E₈) and sedimentation of the nucleocapsid by ultracentrifugation is added to the film. The quantities of additional lipid and supernatant are chosen such that the desired ratio of viral to additional lipid is obtained. The detergent is then removed via treatment with BioBeads or the like as described above.

Generally, the virosomes should resemble a viral envelope in structure and composition as closely as possible. The virosome preparation should generally consist of a relatively uniform population of vesicles in terms of size and protein-to-lipid ratio. Residual detergent should be minimal and not interfere with virosome function. The virosomes should mimic the biological activity of the native viral envelope. Generally, the virosomes should exhibit pH-dependent membrane fusion activity.

Virosomes can also be prepared with viral fusion proteins having different pH sensitivities, derived from, e.g. different influenza virus strains. The different pH sensitivities of the virosome can be taken advantage of to prepare virosome-liposome hybrids that encapsulate and deliver large therapeutic molecules such as DNA that may be difficult to encapsulate directly and with high efficiency in virosomes prepared according to the above protocol. A liposome is first prepared which encapsulates the therapeutic agent with high efficiency. The liposome is then fused with the virosome at the pH of the viral membrane fusion protein having the higher pH threshold for fusion. This results in a virosome-liposome hybrid containing the encapsulated therapeutic agent. The virosome-liposome hybrid is then used to deliver the encapsulated therapeutic agent to the cytosol of cells by fusion triggered at the pH of the viral fusion protein with the lower pH threshold for fusion.

The incorporation of hemagglutinin in reconstituted vesicles is readily assessed by equilibrium density-gradient analysis. The virosome preparation, collected from the discontinuous sucrose gradient, is diluted with HNE and applied to a linear sucrose gradient in HNE (e.g., 10-60% (w/v)) and the gradient centrifuged (e.g., at 170,000 g for 30 hr at 4°), after which fractions are collected and analyzed for protein and phospholipid content. The virosomes appear as a single band, containing both protein and phospholipids. The density of the virosomes will differ depending on the presence of additional lipids. In general, the density will decrease when the ratio of additional lipids to viral lipids increases.

Analysis of influenza virosomes by sodium dodecyl sulfate-polyacrylamide gel electrophoresis (SDS-PAGE) may be performed to confirm that the virosomes contain the hemagglutinin protein. The viral nucleoprotein NP, the matrix protein M1 the minor integral membrane protein of influenza virus, M2, are generally not detectable in such analysis. The virosomes have a protein-to-(phospho)lipid ratio that is similar to the ratio in the solubilization mixture after sedimentation of the nucleocapsid, but which will change when additional lipid is added to the virosome preparation.

Recovery of viral membrane protein and phospholipid in the virosome preparation ranges from 30 to 50% relative to the initially solubilized material. Residual detergent in virosomes prepared according to the above protocol is typically about 7.5 mol % relative to the total virosomal lipid. This level of detergent generally does not significantly affect the fusogenic activity of the virosomes, but residual detergent may have an effect on the capacity of the virosomes to retain low-molecular-weight encapsulated compounds.

Negative-stain electron microscopy (EM) is the most widely applied and accessible technique for assessing the structure and size of virosomes. The staining solution preferably has a neutral pH, so as to avoid acid-induced conformational changes of the hemagglutinin protein. Briefly, a droplet of the virosome suspension, after dialysis against isotonic ammonium acetate buffered to neutral pH with 5 mM HEPES, is applied to a grid with a carbon-coated Formvar film, after glow-discharge of the grid. The specimen is placed upside down for 1 min on a droplet of 2% phosphotungstic acid (PTA) at neutral pH (or, e.g., 1% sodium silicotungstate of neutral pH), drained and dried in air.

Fusion of virosomes with biological or artificial target membranes can be followed with a fluorescent resonance energy transfer assay (RET). In a convenient assay, N-(7-nitrobenz-2-oxa-1,3-diazol-4-yl)phosphatidylethanolamine (N-NBD-PE) is used as a donor probe and N-(lissamine rhodamine B sulfonyl)PE (N-Rh-PE) as the acceptor. A variant of this assay, utilizing the same donor N-NBD-PE but a different acceptor, cholesterol-anthracene-9-carboxylate (CAC), may also be used. Upon fusion of a membrane, labeled with the N-NBD-PE/N-Rh-PE pair, the two fluorophores dilute into the target membrane, resulting in a decrease of their overall surface density and a concomitant decrease of the RET efficiency. This decrease can be followed as an increase of the donor (N-NBD-PE) fluorescence. This assay can be used to assess pH-dependent fusion of influenza virosomes with a membrane, including, e.g., erythrocyte ghosts and BHK cells.

Another in vitro means to assess fusion of virosomes is an excimer assay using pyrene-labeled lipids. Pyrene fluorophores may form excited dimers (excimers) between a probe molecule in the excited state and a probe molecule in the ground state. The fluorescence emission of the excimer is shifted to higher wavelengths by about 100 nm relative to the emission of the monomer. Excimer formation is dependent on the distance between the probe molecules. Thus, coupled to one of the acyl chains of a phospholipid molecule, such as phosphatidylcholine (PC), the pyrene probe provides a sensitive measure of the surface density of the labeled molecules in a lipid bilayer membrane. On fusion of a pyrene-PC-labeled membrane with an unlabeled membrane, the pyrene-PC surface density decrease can be monitored as a reduction of the excimer fluorescence.

The RET probes N-NBD-PE/N-Rh-PE or the Pyrene-PC probe (Molecular Probes, Eugene, OR) are incorporated in the virosomal membrane as follows. The supernatant obtained after solubilization of the viral membrane and sedimentation of the nucleocapsid (see above) is added to a dry film of the probe (10 mol % with respect to the viral lipid). The mixture is lightly shaken to allow mixing of the probe with the viral lipids, and detergent is removed as described above.

Fusion of the labeled virosomes can be conveniently measured using resealed human erythrocyte ghosts as a model biological target membrane system. Alternatively, fusion activity toward liposomes can be assessed, in which case it is important to avoid liposomes consisting primarily of negatively charged phospholipids, such as cardiolipin, as these appear to support a fusion reaction with influenza virus or virosomes, whose characteristics deviate from those of fusion with biological membranes. Fusion with liposomes consisting of a 2:1 mixture of PC and PE (Avanti Polar Lipids, Alabaster, AL), and containing 5 mol % of the ganglioside G_{Dta} or total bovine brain gangliosides (Sigma Chemical Co., St. Louis, MO) serving as sialic acid-containing receptors for the virus/virosomes, provides a convenient assay. Fusion may also be monitored in an on-line fashion using cultured cells as targets. Either endocytic uptake of the virosomes at neutral pH and subsequent fusion from within endosomes or direct fusion with the cell plasma membrane induced by a transient lowering of the extracellular pH may be used.

An alternative to direct assessment of the fusion activity of influenza virosomes is determining their hemolytic activity. The fusion activity of influenza virosomes, produced according to the procedure described above, typically corresponds closely to hemolytic activity, exhibiting a pH dependence identical to that of fusion. Hemolytic activity of influenza virosomes may be determined by, for example, adding the virosomes (the equivalent of 1 nmol of phospholipid, in a volume of 25 µl) to 4 x 10⁷ washed human erythrocytes in 975 µl fusion buffer (135 mM NaCl, 15 mM sodium citrate, 10 mM MES, 5 mM HEPES), set to various pH values. After incubation at 37° for 30 min, the mixture is centrifuged for 3 min at 1350 g. Lysis of erythrocytes is quantified by the measurement of absorbance of the hemoglobin in the supernatant at 541 nm. Maximal hemolysis is determined after lysis of the erythrocytes in distilled water.

Additional components may be added to the virosomes to target the virosomes to specific cell types. For example, the virosomes can be conjugated to monoclonal antibodies that bind to epitopes present only on specific cell types. For example, monoclonal antibodies may bind specifically to cancer-related antigens providing a means for targeting the virosomes following systemic administration. Alternatively, ligands that bind surface receptors of the target cell types may also be bound to the virosomes. Other means for targeting liposomes may also be employed in the present invention.

The fusogenic virosomes are employed to carry therapeutic compounds for introduction into cells. As used herein, "therapeutic compound" is meant to indicate a synthetic compound suitable for therapeutic use. "Therapeutic compound" means nucleic acids (antisense, DNA). "Synthetic compounds" are compounds that are not naturally occurring or compounds that are isolated from the environment in which they naturally occur.

The therapeutic compound may be carried in the aqueous interior of the virosome or in the lipid membrane of the virosome. A variety of therapeutic compounds may be carried in the virosomes of the present invention. The virosomes provide a means for facilitated entry of the therapeutic compounds into the cells.

Particularly useful is encapsulation of therapeutic compounds that are active within the cytoplasm of host cells. Such compounds include, e.g., DNA encoding proteins or peptides operably linked to a promoter active in the host cell, RNA encoding a protein or peptide, nucleic acids such as antisense oligonucleotides (as described in, e.g., WO 93/09813 and WO 93/01286 and ribozymes (e.g., U.S. Patent Nos. 4,987,071, 5,254,678, and WO 94/26877). The therapeutic compounds are delivered into the host cell cytoplasm upon fusion of the virosome with the endosome or plasma membrane.

The therapeutic compounds will generally be foreign to the host. By "foreign," it is meant a compound that is not naturally present in the host. Alternatively, the therapeutic compound may not be foreign to the host. The compound may naturally occur within the host. For example, nucleic acids encoding a naturally occurring protein may be introduced into host cells to increase expression of the protein in the cells. The nucleic acid can be either DNA or RNA. For expression, the nucleic acid will typically comprise at least the following operably linked elements: a transcriptional promoter, a gene encoding the desired therapeutic protein, and a transcriptional terminator.

Therapeutic compounds may be incorporated into the virosome at the time of virosome preparation. Typically, the therapeutic compound is added to the lipid/hemagglutinin-containing solution following removal of the nucleocapsid. Alternatively, the therapeutic compound is encapsulated in a virosome-liposome hybrid by initial encapsulation of the compound in a liposome, followed by fusion of the liposome with a virosome containing two hemagglutinins with different pH thresholds for fusion, as outlined above.

For administration to host cells the virosomes are carried in a pharmaceutically acceptable carrier. Many pharmaceutically acceptable carriers may be employed in the compositions of the present invention. Generally, normal buffered saline (135-150 mM NaCl) will be employed as the pharmaceutically acceptable carrier, but other suitable carriers will suffice. These compositions may be sterilized by conventional liposomal sterilization techniques, such as filtration. The compositions may contain pharmaceutically acceptable auxiliary substances as required to approximate physiological conditions, such as pH adjusting and buffering agents, tonicity adjusting agents and the like, for example, sodium acetate, sodium lactate, sodium chloride, potassium chloride, calcium chloride, etc.

The concentration of virosomes in the carrier may vary. Generally, the concentration will be about 20-200 mg/ml, usually about 50-150 mg/ml, and most usually about 75-125 mg/ml, e.g., about 100 mg/ml. Persons of skill may vary these concentrations to optimize treatment with different virosome components or for particular patients. For example, the concentration may be increased to lower the fluid load associated with treatment. This may be particularly desirable in patients having atherosclerosis-associated congestive heart failure or severe hypertension.

The methods for introducing therapeutic compounds into cells of a host generally comprise contacting the cells of the host with a virosome containing the therapeutic compound, wherein the virosome has a membrane and an aqueous interior, and a viral membrane fusion protein, e.g., influenza hemagglutinin, is contained in the membrane. The host may be a variety of animals, including humans, non-human primates, avian species, equine species, bovine species, swine, lagomorpha, rodents, and the like.

The cells may be contacted by in vivo administration of the virosomes or ex vivo contacting of the virosomes to the cells. In vivo contact is obtained by administration of the virosomes to host. The virosomes may be administered in many ways. These include parenteral routes of administration, such as intravenous, intramuscular, subcutaneous, and intraarterial. Generally, the virosomes will be administered intravenously or via inhalation. Often, the virosomes will be administered into a large central vein, such as the superior vena cava or inferior vena cava, to allow highly concentrated solutions to be administered into large volume and flow vessels. The virosomes may be administered intraarterially following vascular procedures to deliver a high concentration directly to an affected vessel. The virosomes may also be administered topically. In some instances, the virosomes may be administered orally or transdermally. The virosomes may also be incorporated into implantable devices for long term release following placement.

As described above, the virosomes are typically administered intravenously or via inhalation in the methods of the present invention. Often multiple treatments will be given to the patient. The dosage schedule of the treatments will be determined by the disease and the patient's condition. Standard treatments with therapeutic compounds that are well known in the art may serve as a guide to treatment with virosomes containing the therapeutic compounds. The duration and schedule of treatments may be varied by methods well known to those of skill.

The dose of virosomes of the present invention may vary depending on the clinical condition and size of the animal or patient receiving treatment. The standard dose of the therapeutic compound when not encapsulated may serve as a guide to the dose of the virosome-encapsulated compound. The dose will typically be constant over the course of treatment, although the dose may vary in some instances. Standard physiological parameters may be assessed during treatment that may alter the dose of the virosomes.

The following examples are offered by way of illustration and not limitation.

### EXAMPLE I

This Example illustrates the fusogenicity of virosomes. Pyrene-PC-labeled influenza virosomes were fused with human erythrocyte ghosts.

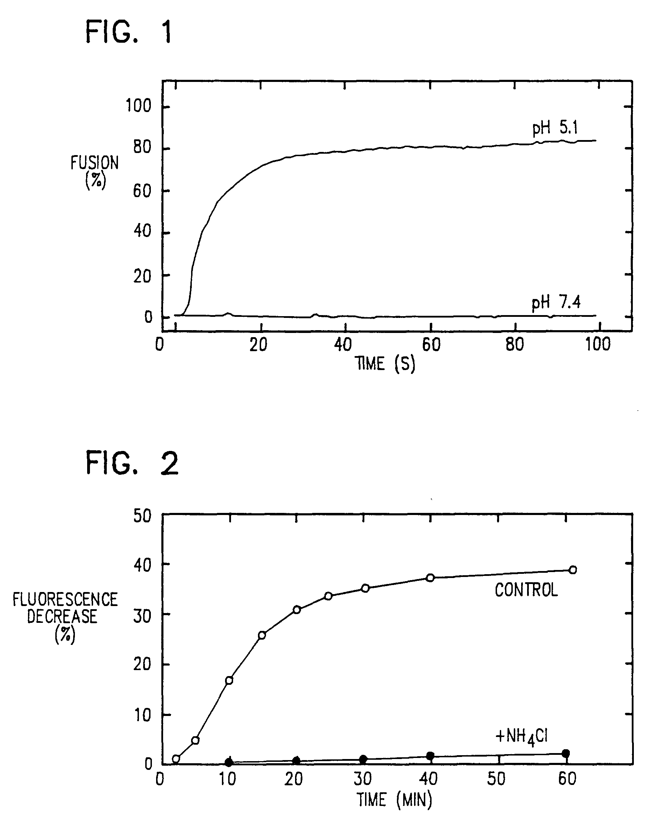

The preparation of the virosomes was as described above. Pyrene-PC was incorporated, as also described above, to 10 mole% relative to the viral lipid. Fusion was measured after prebinding of the virosomes to erythrocyte ghosts for 15 minutes at 4°C and pH 7.4. The suspension was placed in a fusion medium of 135 mM NaCl, 15 mM sodium citrate, 10 mM MES, and 5 mM HEPES at 37°C in a fluorimeter cuvette. At time zero the pH in the cuvette was adjusted to 5.1 or maintained at 7.4. Fusion was detected as a decrease of the pyrene excimer fluorescence. Fig. 1 illustrates that the fusion of the virosomes is strictly dependent on low pH.

Intracellular fusion of virosomes to BHK-21 cells was also assessed. Pyrene-PC-labeled virosomes were allowed to bind to the plasma membrane of trypsinized BHK-21 cells at 4° for 1 hr in Hank's/HEPES buffer (137 mM NaCl, 5.4 mM KCl, 0.44 mM KH₂PO₄, 0.41 mM MgSO₄, 0.40 mM MgCl₂, 1.3 mM CaCl₂, 5.6 mM glucose, and 10 mM HEPES, pH 7.4). After washing, the virosome-cell mixture was subsequently injected into the fluorescence cuvette containing prewarmed (37°) Hanks/HEPES buffer. Fusion was monitored as a decrease of the pyrene excimer fluorescence. As shown in Fig. 2, after a lag phase of 3-4 min, explained by the time required for the virosomes to reach the endosomes, the excimer fluorescence decreased in 1 hr to less than 60% of the initial value, indicating that over 40% of the cell-associated virosomes had fused. Under the conditions of the experiments, this corresponds to approximately 1500 virosomes fused per cell. When performed in the presence of 20mM NH₄Cl, an inhibitor of endosomal acidification, the fusion was blocked (Fig. 2).

### EXAMPLE II

This Example demonstrates delivery of Diphtheria toxin A (DTA) to the cytoplasm of mammalian cells. DTA is the toxic subunit of Diphtheria toxin and very efficiently inhibits cellular protein synthesis by ADP-ribosylation of elongation factor 2. However, when added to cells, DTA is not toxic as it can not bind to cellular receptors for the toxin, a capacity that is located on the B subunit of the intact toxin. Isolated DTA was encapsulated in virosomes during their preparation. This DTA was delivered to the cytoplasm of BHK-21 cells via endocytic uptake of the virosomes and fusion at the level of the endosomes.

The encapsulation of DTA in the virosomes was according to the procedure described for encapsulation of gelonin set forth in Example 3 below. DTA was dissolved at a concentration of about 0.5 mg/ml (25 µM) in the supernatant after ultracentrifugation of the detergent-solubilized virus during virosome preparation. The virosomes were incubated with monolayers of BHK-21 cells at 4°C for 1 hr. The cells were washed as described for gelonin and cultured for 6 hr. Protein synthesis was measured by incorporation of ³H-leucine in TCA-precipitable material.

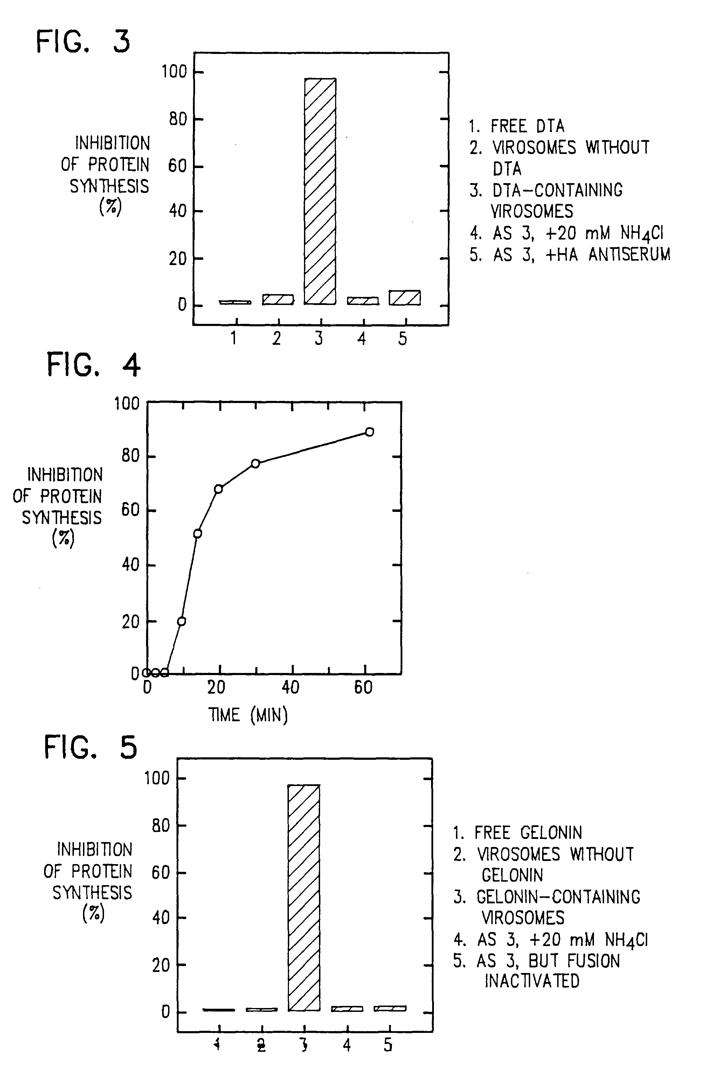

As shown in Fig. 3, DTA encapsulated in fusogenic virosomes induced complete inhibition of the cellular protein synthesis (bar 3). In contrast, free DTA (bar 1) or empty virosomes (bar 2) had no effect on protein synthesis. The effect of virosome-encapsulated DTA was blocked completely with NH₄Cl (bar 4), or by an antiserum against the hemagglutinin (bar 5).

### EXAMPLE III

This Example demonstrates introduction of a protein, gelonin, into mammalian cells. Large amounts of gelonin were introduced into the cells with virosome compositions of the present invention.

Gelonin is a single chain protein, found in the seeds of *Gelonlum multiflorum* which can catalytically inactivate 60 S ribosome subunits (Stirpe et al., A. J. Biol. Chem., 255:6947 (1980)). However, gelonin lacks the ability to bind to cell surfaces, and is therefore essentially nontoxic when added exogenously to living cells.

Gelonin was encapsulated in the aqueous interior of influenza virosomes. Purified influenza virus from strain NIB24 (equivalent to about 1.5 µmol membrane phospholipid, representing about 5 mg of protein) was diluted in a buffer of neutral pH (*e.g.* 5.0 mM Hepes, pH 7.4, containing 0.15 M NaCl) and sedimented at 4°C via ultracentrifugation. To the pellet 0.7 ml of a solution of 100 mM C₁₂E₈ in the same buffer was added. The pellet was resuspended, and the virus solubilized, by flushing the solution several times through a 25-gauge needle, followed by a 15 min incubation at room temperature, and a repeated flushing through a 25-gauge needle. Almost complete solubilization of the viral membrane proteins and lipids was achieved with this method.

The viral nucleocapsid was removed by ultracentrifugation at 4°C. The supernatant containing the viral hemagglutinin was added to 0.5 mg lyophilized gelonin. The clear supernatant was transferred to a vial containing 0.18 g wet Biobeads SM-2. This hydrophobic resin bound the C₁₂E₈, thus removing it from the solution. The suspension was gently shaken (to ensure intimate contact between beads and suspension) for 90 min, followed by addition of 0.35 g wet Biobeads and another extraction period of 60 min. The suspension was removed from the beads and applied on top of a discontinuous sucrose gradient, composed of 0.5 ml 40% (w/v) sucrose and 2.0 ml 10% (w/v) sucrose in 5.0 mM Hepes (pH 7.2) containing 0.15 M NaCl. The gradients were centrifuged for 90 min. at 170,000xg and 4°C. The virosomes were collected from the interface of the two sucrose-containing layers.

The gelonin-containing virosomes were administered to BHK-21 cells. Virosomes at a dose of 5 µM phospholipid were allowed to bind to a monolayer of 5x10⁴ BHK-21 cells for 1 hour at 4°C. After washing with ice-cold buffer, internalization was induced by addition of buffer (pH 7.4) warmed to 37°C. At 2, 5, 10, 15, 30 and 60 min following the addition of warm buffer, the buffer was replaced by culture medium containing 20 mM NH₄Cl. In the control, medium with NH₄Cl was present from time zero. After culturing for 16 hours, the level of protein synthesis was determined by incorporation of ³H-Leucine. Levels of protein synthesis are expressed relative to levels obtained with untreated cells.

No inhibition of protein synthesis was noted up to 5 min after induction of internalization of the gelonin-containing virosomes (Fig. 4). This time interval corresponds well with the time required for the first virosomes to reach the endosomal compartment as described above (see Fig. 2). After further incubation, protein synthesis was progressively inhibited. After 1 h of internationalization, protein synthesis was almost completely blocked.

The data presented in Fig. 5 demonstrate that the inhibition of protein synthesis is due to a hemagglutinin-mediated membrane fusion event between gelonin-containing virosomes and the limiting membrane of the endosomal compartment. BHK-21 cells were incubated with free gelonin (incubated for 3 hr at 37°C, bar 1) or unloaded virosomes (virosomes without gelonin pre-bound for 1 hr at 4°C and internalized for 1 hr at 37°C, bar 2) as controls. Measurable inhibition of protein synthesis did not occur with incubation with either free gelonin or unloaded virosomes. Only following internalization of gelonin-containing virosomes (pre-bound for 1 hr period at 4°C and internalized for 1 hr at 37°C) was protein synthesis blocked (bar 3). Moreover, internalization in the presence of 20 mM NH₄Cl abolished the inhibition of protein synthesis (bar 4), and pre-exposure of virosomes to pH 5.4 at 37°C for 5 min prior to binding, which is known to inactivate the membrane fusion activity, abolished inhibition of protein synthesis following internalization at 37°C (bar 5). In conclusion, inhibition of protein synthesis in living cells by gelonin encapsulated in virosomes is solely mediated by HA-specific fusion of the virosomal membrane with the limiting membrane of the endosomal compartment.

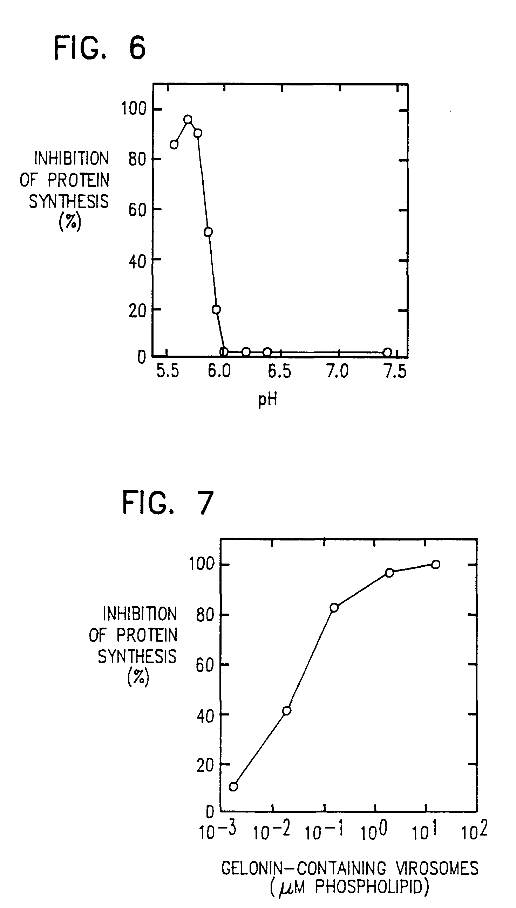

Fig. 6 demonstrates that virosomes can fuse with the cell plasma membrane mediating the delivery of gelonin. Gelonin-containing virosomes prepared as above were allowed to bind to a monolayer of BHK-21 cells for 1 hr at 4°C. The virosomes were administered at a dose of 5.0 µM phospholipid. After incubation the cells were washed with ice-cold buffer and fusion was induced by addition of buffer at 37°C. Acidity of the buffer was adjusted to pH 5.5, 5.6, 5.7, 5.8, 5.9, 6.0, 6.2, 6.4 and 7.4 in different experiments. After incubation for 2 min at 37°C, the buffer was replaced by culture medium containing 20 mM NH₄Cl. After 16 hr culture at 37°C, inhibition of protein synthesis was determined relative to untreated cells.

Following incubation at pH 5.6, protein synthesis was almost completely inhibited. Incubation at pH 6.0 did not produce inhibition of protein synthesis. pH 6.0 and pH 5.6 correspond closely to the threshold and optimum pH, respectively, for obtaining lipid mixing of this strain of virus with erythrocyte ghosts. Thus, delivery of gelonin, encapsulated in virosomes by fusion with the plasma membranes of living cells is also dependent on low-pH-dependent activation of the fusion activity of HA.

The efficiency of fusion-mediated delivery of gelonin was evaluated. At the lipid concentration at which the virosomes are produced about 0.7% of the aqueous phase of the liposome-forming solution will be trapped in the lumen of the virosomes. It was estimated that approximately 5 molecules of gelonin were encapsulated per virosome. At doses of 20 µM phospholipid, BHK-21 cells internalize approximately 3,000 virosomes per cell, when the virosomes are allowed to prebind to the cells in the cold and the unbound virosomes are washed away before the virosome internalization is induced. About 40% of the 3,000 internalized virosomes fuse with the endosomal membranes (Fig. 2). Thus, under these conditions 6,000 copies of molecules having the size of gelonin may be delivered to the cytosol of a single cell. When the virosome prebinding step in the cold and the subsequent removal of unbound virosomes are not carried out, but instead the total amount of virosomes is allowed to interact with the cells for the 60 min at 37°C, the relative cellular internalization of the virosomes is enhanced about 20-fold. This demonstrates that under these conditions more than 100,000 copies of a molecule such as gelonin can be delivered per cell.

Fig. 7 illustrates titration of gelonin-mediated inhibition of protein synthesis to a level corresponding to a single virosome fusing per cell. When the concentration of virosomes was reduced to 0.020 µM phospholipid (corresponding with about 2-3 virosomes fusing per cell) inhibition of protein synthesis was 42%. Based on the assumptions that virosome internalization by the cells follows a Poissonian distribution and that cells either die (100% inhibition of protein synthesis) or survive (0% inhibition of protein synthesis) it can be estimated that fusion of approximately 3 virosomes per cell (delivery of about 15 copies of gelonin per cell) is lethal. This is close to the minimally possible number and, thus, supports the belief that delivery of encapsulated macromolecules via fusion-active virosomes is a highly efficient way of introducing these substances into the cytoplasm of living cells.

### EXAMPLE IV

This Example demonstrates the delivery of DNA into BHK-cells in vitro with virosomes and the expression of the DNA in the cells. The plasmid pCMV β-gal was complexed to virosomes containing positively charged phospholipid DODAC, and DNA was also encapsulated in virosomes after condensation with polylysine to form a particle that was enclosed within the virosome.

For formation of the virosome-DNA complex, virosomes were prepared containing different amounts of DODAC. The plasmid pCMV β-gal was complexed to virosomes containing 0, 10, 20, 30, 40 or 50 mol % DODAC (% of original total phospholipids in the virus), as follows. The indicated amount of DODAC was either lyophilized or dried under a stream of N₂ followed by vacuum for 2 hours and dissolved in a small volume of 200 mM C₁₂E₈. Virosomes were prepared as described in Example III, except the 0.7 ml supernatant (after ultracentrifugation in the step to remove the nucleocapsid) containing the viral hemagglutinin and viral lipids was added to vials containing the different amounts of DODAC. The detergent was removed with BioBeads SM-2 and the virosomes isolated on the discontinuous sucrose gradient as described above. These virosomes (100 nmol total phospholipid) were incubated with 30 µg pCMV β-gal in a final vol. of 200 µl PBS for 30 min at RT. The virosome-DNA complexes were added to a monolayer of BHK-cells in 48-well plates (40 µl/well) and allowed to bind for 30 min at 37°C. After this time the culture medium was added and the cells incubated for 48 hrs at 37°C. The following controls were used: i) plasmid without virosomes, ii) virosomes pre-exposed to pH 5.4 at 37° for 15 min prior to formation of the DNA-complex, and iii) culture medium containing 20 mM NH₄Cl. The exposure of virosomes to acidic pH is known to inactivate the membrane fusion activity of virus and virosomes. Ammonium chloride in the cell medium increases the normally acidic pH in the endosomal compartment thereby preventing fusion of the virosomes with the endosomal membrane.

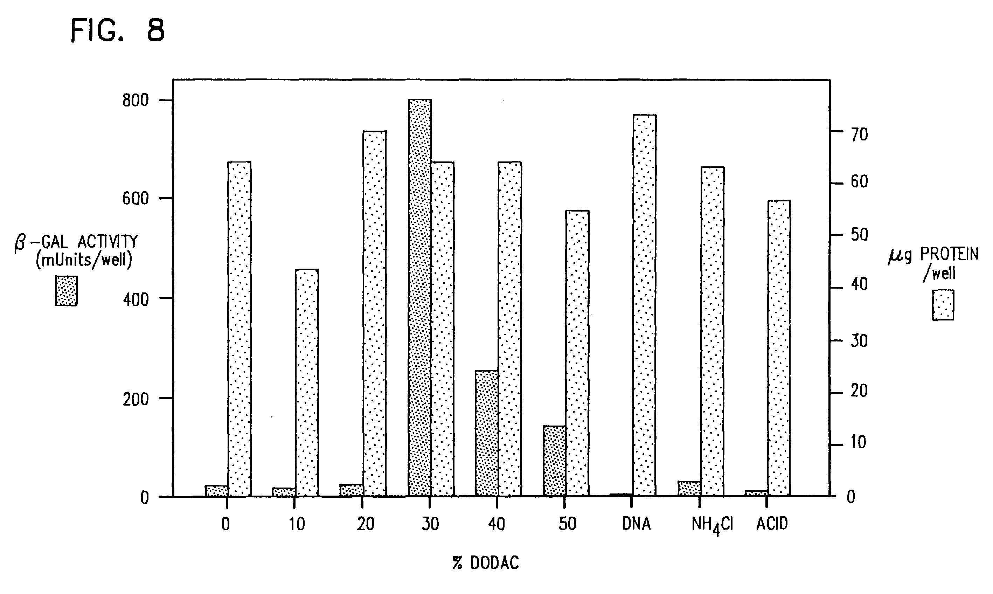

Expression of β-gal in BHK-cells is a direct measure for the delivery of the plasmid to the cell and its incorporation into the nucleus. After a 48 hr incubation the cells were either stained for β-gal activity and photographed or lysed and the protein content and β-gal activity determined. The β-gal activities obtained (expressed in munits/µg protein) by treatment with the DNA complexed to virosomes containing different amounts of DODAC are shown in Fig. 8. Excellent transfection was obtained with virosomes containing 30 mol % DODAC. Transfection was also obtained with virosomes containing 40 and 50 mol % DODAC, but these preparations showed toxic effects on the BHK-cells. No transfection was observed when DNA by itself was added to the cells. Transfection was also abolished when the virosomes were pre-exposed to acidic pH, or when ammonium chloride was used in the cell medium to increase the normally acidic pH in the endosomal compartment. The acid inactivation and the NH₄Cl data indicate that the delivery of the DNA complexed to virosomes was the result of hemagglutinin-mediated fusion in the endosomal compartment.

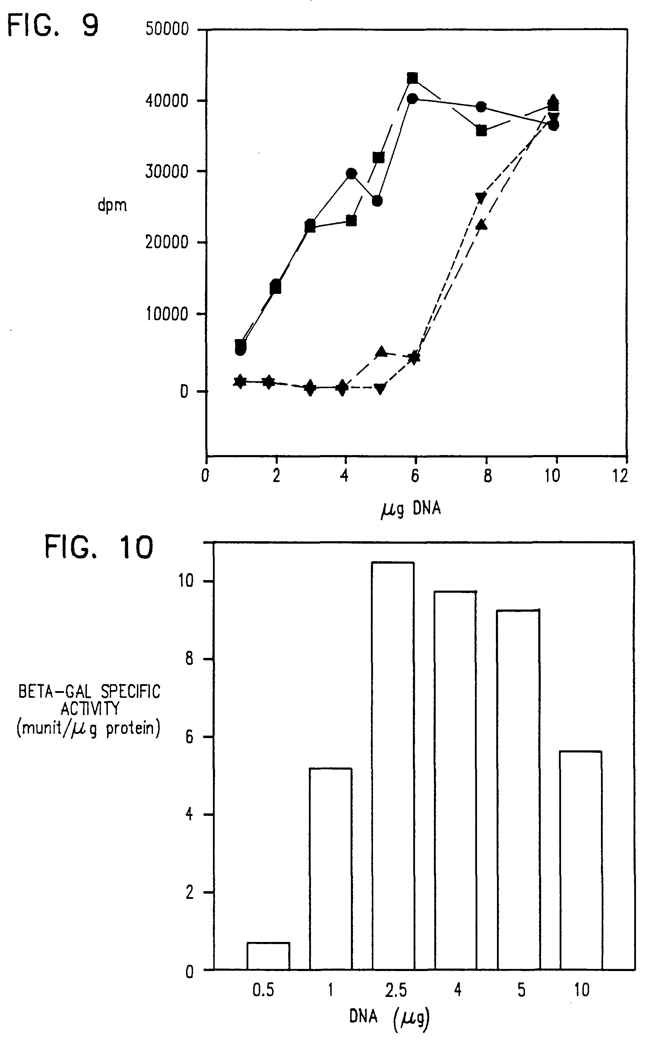

The DNA binding capacity of the virosomes containing 30 mol % DODAC was then determined. Different amounts of ³H-pCMVβ-gal were added to virosomes (20 nmol total phospholipids) and incubated at RT. After 30 min the virosomes were centrifuged (Beckman Microfuge) for 2 min and the radioactivity determined in the supernatant and in the pellet. The pellet was washed two times with HBS-buffer and dissolved in 200 mM C₁₂E₈ prior to the radioactivity measurement. The radioactivity determined in the pellet (●, ■) and in the supernatant (▲, ▼) of two independent experiments are plotted as a function of DNA added to the virosomes (Fig. 9). Binding capacity for virosomes with 20 nmol total phospholipid was reached with 6 µg DNA.

The amount of DNA required for optimum transfection of BHK-cells is shown in Fig. 10. Virosomes containing 30 mol % DODAC were complexed with pCMV β-gal (3 µg DNA/10 nmol total phospholipid in virosomes) and added to the cell monolayer to give the amount of DNA per well as indicated in Fig. 10. β-gal activity was determined in the cell lysate as units per µg protein and plotted as a function of DNA added with the virosome complex. Optimal transfection was obtained with 2.5 µg DNA per well using 48 well plates.

## Claims

1. A pharmaceutical composition for introducing a therapeutic nucleic acid into a cell of a host, comprising a virosome having a membrane and an aqueous interior, wherein the virosome membrane comprises a viral membrane fusion protein and a cationic lipid suitable for complexing said nucleic acid to the virosome, the therapeutic nucleic acid complexed to said virosome, and a pharmaceutically acceptable carrier.

2. The pharmaceutical composition of claim 1, wherein the therapeutic nucleic acid is contained in the aqueous interior of the virosome.

3. The pharmaceutical composition of claim 1, wherein the therapeutic nucleic acid is contained in the membrane of the virosome.

4. The pharmaceutical composition of claim 1, wherein the therapeutic nucleic acid is DNA or antisense RNA.

5. The pharmaceutical composition of claim 1, wherein the therapeutic nucleic acid is foreign to the host.

6. The pharmaceutical composition of claim 1, wherein the virosome membrane comprises phosphatidylserine or octaethyleneglycol monododecyl ether.

7. The pharmaceutical composition of claim 6, wherein the virosome membrane further comprises cholesterol.

8. The pharmaceutical composition of claim 1, wherein the viral membrane fusion protein is influenza hemagglutinin.

9. The pharmaceutical composition of claim 8, wherein the influenza hemagglutinin is prepared from influenza 3A virus.

10. The pharmaceutical composition of claim 1, wherein the cationic lipid is DODAC (N,N-dioleyl-N,N-dimethylammonium chloride).

11. The pharmaceutical composition of claim 10, wherein the DODAC is present in a concentration of 25-45 % phospholipid content.

12. The use of a virosome in the preparation of a medicament for introducing a therapeutic nucleic acid into a cell in a host, comprising a virosome having a membrane and an aqueous interior, wherein the virosome membrane comprises a viral membrane fusion protein and a cationic lipid suitable for complexing said nucleic acid to the virosome, the therapeutic nucleic acid complexed to said virosome, and a pharmaceutically acceptable carrier.

13. The use of claim 12, wherein the nucleic acid molecule comprises the following operably linked elements:
a transcriptional promotor,
a gene encoding a therapeutic protein, and
a transcriptional terminator.

14. The use of claim 12, wherein the therapeutic nucleic acid is foreign to the host.

15. The use of claim 12, wherein the virosome membrane comprises phosphatidylserine or octaethyleneglycol monododecyl ether.

16. The use of claim 15, wherein the virosome further comprises cholesterol.

17. The use of claim 12, wherein the medicament is suitable for topical administration.

18. The use of claim 12, wherein the medicament is suitable for parenteral administration.

19. The use of claim 12, wherein the viral membrane fusion protein is influenza hemagglutinin protein.

20. The use of claim 19, wherein the hemagglutinin protein is derived from influenza A.

21. A method for preparing a composition for introducing a nucleic acid into a cell of a host, comprising:
condensing the nucleic acid with polylysine to form nucleic acid particles,
combining the nucleic acid particles with a mixture of a viral membrane fusion protein, lipid and detergent, and
removing the detergent to form a virosome having a membrane and an aqueous interior, the viral membrane fusion protein being contained in the membrane, thereby passively encapsulating the nucleic acid particles in the interior of the virosome.

## Patentansprüche

1. Pharmazeutische Zusammensetzung zum Einbringen einer therapeutischen Nukleinsäure in eine Wirtszelle, enthaltend
ein Virosom mit einer Membran und einem wässrigen Inneren, wobei die Virosom-Membran ein virales Membranfusionsprotein und ein kationisches Lipid enthält, das zum Komplexieren der Nukleinsäure mit dem Virosom geeignet ist,
die therapeutische Nukleinsäure, die mit dem Virosom komplexiert ist, und
einen pharmazeutisch akzeptablen Träger.

2. Pharmazeutische Zusammensetzung nach Anspruch 1
bei der die therapeutische Nukleinsäure in dem wässrigen Inneren des Virosoms enthalten ist.

3. Pharmazeutische Zusammensetzung nach Anspruch 1,
bei der die therapeutische Nukleinsäure in der Membran des Virosoms enthalten ist.

4. Pharmazeutische Zusammensetzung nach Anspruch 1,
bei der die therapeutische Nukleinsäure DNA oder antisense RNA ist.

5. Pharmazeutische Zusammensetzung nach Anspruch 1,
bei der die therapeutische Nukleinsäure wirtsfremd ist.

6. Pharmazeutische Zusammensetzung nach Anspruch 1,
bei der die Virosom-Membran Phosphatidylserin oder Octaethylenglykolmonododecylether enthält.

7. Pharmazeutische Zusammensetzung nach Anspruch 6,
bei der die Virosom-Membran ferner Cholesterin enthält.

8. Pharmazeutische Zusammensetzung nach Anspruch 1,
bei der das virale Membranfusionsprotein Influenza-Hämagglutinin ist.

9. Pharmazeutische Zusammensetzung nach Anspruch 8,
bei der das Influenza-Hämagglutinin aus dem Influenza-3A-Virus hergestellt ist.

10. Pharmazeutische Zusammensetzung nach Anspruch 1,
bei der das kationische Lipid DODAC (N, N-Dioleyl-N,N-dimethylammoniumchlorid) ist.

11. Pharmazeutische Zusammensetzung nach Anspruch 10,
bei der das DODAC in einer Konzentration von 25 bis 45 % des Phospholipidgehaltes vorhanden ist.

12. Verwendung eines Virosoms zur Herstellung eines Medikamentes zum Einbringen einer therapeutischen Nukleinsäure in eine Wirtszelle, enthaltend
ein Virosom mit einer Membran und einem wässrigen Inneren, wobei die Virosom-Membran ein virales Membranfusionsprotein und ein kationisches Lipid enthält, das zum Komplexieren der Nukleinsäure mit dem Virosom geeignet ist,
die therapeutische Nukleinsäure, die mit dem Virosom komplexiert ist, und
einen pharmazeutisch akzeptablen Träger.

13. Verwendung nach Anspruch 12,
bei der das Nukleinsäure-Molekül die folgenden funktionsfähig gebundenen Elemente enthält:
einen Transkriptionspromotor,
ein Gen, das ein therapeutisches Protein kodiert, und
einen Transkriptionsterminator.

14. Verwendung nach Anspruch 12,
bei der die therapeutische Nukleinsäure wirtsfremd ist.

15. Verwendung nach Anspruch 12,
bei der die Virosom-Membran Phosphatidylserin oder Octaethylenglykolmonododecylether enthält.

16. Verwendung nach Anspruch 15,
bei der das Virosom ferner Cholesterin enthält.

17. Verwendung nach Anspruch 12,
bei der das Medikament für eine topische Verabreichung geeignet ist.

18. Verwendung nach Anspruch 12,
bei der das Medikament für eine parenterale Verabreichung geeignet ist.

19. Verwendung nach Anspruch 12,
bei der das virale Membranfusionsprotein ein Influenza-Hämagglutinin-Protein ist.

20. Verwendung nach Anspruch 19,
bei der das Hämagglutinin-Protein aus Influenza-A stammt.

21. Verfahren zum Herstellen einer Zusammensetzung zum Einbringen einer Nukleinsäure in eine Wirtszelle, umfassend:
Kondensieren der Nukleinsäure mit Polylysin zur Bildung von Nukleinsäure-Partikeln,
Zusammenbringen der Nukleinsäure-Partikel mit einer Mischung aus einem viralen Membranfusionsprotein, einem Lipid und einem Detergens, und
Entfernen des Detergens, um ein Virosom mit einer Membran und einem wässrigen Inneren zu bilden, wobei das virale Membranfusionsprotein in der Membran enthalten ist,
wodurch die Nukleinsäure-Partikel im Inneren des Virosoms passiv eingeschlossen werden.

## Revendications

1. Composition pharmaceutique pour introduire un acide nucléique thérapeutique dans une cellule d'un hôte, comprenant un virosome ayant une membrane et un compartiment intérieur aqueux, dans laquelle la membrane du virosome comprend une protéine de fusion de membrane virale et un lipide cationique apte à la complexation dudit acide nucléique au virosome, l'acide nucléique thérapeutique complexé audit virosome et un support pharmaceutiquement acceptable.

2. Composition pharmaceutique suivant la revendication 1, dans laquelle l'acide nucléique thérapeutique est présent dans le compartiment intérieur aqueux du virosome.

3. Composition pharmaceutique suivant la revendication 1, dans laquelle l'acide nucléique thérapeutique est présent dans la membrane du virosome.

4. Composition pharmaceutique suivant la revendication 1, dans laquelle l'acide nucléique thérapeutique consiste en un ADN ou un ARN antisens.

5. Composition pharmaceutique suivant la revendication 1, dans laquelle l'acide nucléique thérapeutique est étranger à l'hôte.

6. Composition pharmaceutique suivant la revendication 1, dans laquelle la membrane du virosome comprend de la phosphatidylsérine ou de l'éther monododécylique d'octa-éthylène-glycol.

7. Composition pharmaceutique suivant la revendication 6, dans laquelle la membrane du virosome comprend en outre du cholestérol.

8. Composition pharmaceutique suivant la revendication 1, dans laquelle la protéine de fusion de membrane virale est l'hémagglutinine de virus grippal.

9. Composition pharmaceutique suivant la revendication 8, dans laquelle l'hémagglutinine de virus grippal est préparée à partir du virus grippal 3A.

10. Composition pharmaceutique suivant la revendication 1, dans laquelle le lipide cationique consiste en DODAC (chlorure de N,N-dioléyl-N,N-diméthyl-ammonium).

11. Composition pharmaceutique suivant la revendication 10, dans laquelle le DODAC est présent à une concentration de 25 à 45 % de la teneur en phospholipide.

12. Utilisation d'un virosome dans la préparation d'un médicament destiné à introduire un acide nucléique thérapeutique dans une cellule d'un hôte, comprenant un virosome ayant une membrane et un compartiment intérieur aqueux, dans laquelle la membrane du virosome comprend une protéine de fusion de membrane virale et un lipide cationique apte à la complexation dudit acide nucléique au virosome, l'acide nucléique thérapeutique complexé audit virosome, et un support pharmaceutiquement acceptable.

13. Utilisation suivant la revendication 12, dans laquelle la molécule d'acide nucléique comprend les éléments suivants liés de manière fonctionnelle :
un promoteur de transcription,
un gène codant pour une protéine thérapeutique, et
un terminateur de transcription.

14. Utilisation suivant la revendication 12, dans laquelle l'acide nucléique thérapeutique est étranger à l'hôte.

15. Utilisation suivant la revendication 12, dans laquelle la membrane du virosome comprend de la phosphatidylsérine ou de l'éther monododécylique d'octa-éthylène-glycol.

16. Utilisation suivant la revendication 15, dans laquelle le virosome comprend en outre du cholestérol.

17. Utilisation suivant la revendication 12, dans laquelle le médicament est apte à l'administration topique.

18. Utilisation suivant la revendication 12, dans laquelle le médicament est apte à l'administration parentérale.

19. Utilisation suivant la revendication 12, dans laquelle la protéine de fusion de membrane virale est l'hémagglutinine de virus grippal.

20. Utilisation suivant la revendication 19, dans laquelle la protéine hémagglutinine est dérivée du virus grippal A.

21. Procédé pour la préparation d'une composition destinée à l'introduction d'un acide nucléique dans une cellule d'un hôte, comprenant les étapes consistant :
à condenser l'acide nucléique avec de la polylysine pour former des particules d'acide nucléique,
à combiner les particules d'acide nucléique avec un mélange d'une protéine de fusion de membrane virale, d'un lipide et d'un détergent, et
à éliminer le détergent pour former un virosome comprenant une membrane et un compartiment intérieur aqueux, la protéine de fusion de membrane virale étant présente dans la membrane, en encapsulant ainsi passivement les particules d'acide nucléique à l'intérieur du virosome.
